# EUROPEAN PATENT APPLICATION

(11) **EP 3 742 167 A1**
(43) Date of publication of application: **25.11.2020**
(21) Application number: 19741906.2
(22) Date of filing: 16.01.2019
(51) Int. Cl.: G01N 33/68, G01N 33/53

(54) **COMPOSITION FOR SCREENING ONGOING PROGRESS OF ALZHEIMER'S DISEASE BY USING BETA AMYLOID OLIGOMER IN NASAL DISCHARGE SPECIMEN AND METHOD FOR SCREENING ONGOING PROGRESS OF ALZHEIMER'S DISEASE BY USING SAME**

(30) Priority: 16.01.2018 KR 20180005761
(71) Applicant: Daegu Gyeongbuk Institute Of Science and Technology, Daegu 42988 (KR)
(72) Inventor: MOON, Che Il, Daegu 42060 (KR); YOO, Seung Jun, Gyeongsangbuk-do 38612 (KR); SON, Go Woon, Daegu 42616 (KR); LEE, Yeong Bae, Seoul 06279 (KR); CHANG, Keun A, Seoul 07987 (KR)
(74) Representative: Roos, Peter
(86) International application number: PCT/KR2019/000644
(87) International publication number: WO 2019/143125

(57) **Abstract**

The present invention relates to a composition for screening ongoing progress of Alzheimer's disease using beta amyloid of a nose discharge sample and a method of screening ongoing progress of Alzheimer's disease using the same, more specifically, to a composition and a kit for screening ongoing progress of Alzheimer's disease, comprising an agent measuring an expression level of a 56 kDa beta amyloid oligomer and a 72 kDa beta amyloid oligomer in an nose discharge sample, and a method of screening ongoing progress of Alzheimer's disease comprising measuring the expression level of the 56 kDa and 72 kDa beta amyloid oligomers in an nose discharge sample.

## Description

### [Technical Field]

This application claims priority of Korean Patent Application No. 10-2018-0005761 filed on January 16, 2018 and is hereby incorporated herein by reference in its entirety.

The present invention relates to a composition for screening ongoing progress of Alzheimer's disease using beta amyloid of a nose discharge sample and a method of screening ongoing progress of Alzheimer's disease using the same, more specifically, to a composition and a kit for screening ongoing progress of Alzheimer's disease, comprising an agent measuring an expression level of a 56 kDa beta amyloid oligomer and a 72 kDa beta amyloid oligomer in an nose discharge sample, and a method of screening ongoing progress of Alzheimer's disease comprising measuring the expression level of the 56 kDa and 72 kDa beta amyloid oligomers in an nose discharge sample. Also, the present invention relates to a method of screening a therapeutic agent for mild or moderate Alzheimer's disease comprising measuring an expression level of 56 kDa and 72 kDa beta amyloid oligomers in a nose discharge sample, and a kit for screening a therapeutic agent for mild or moderate Alzheimer's disease comprising measuring an expression level of 56 kDa and 72 kDa beta amyloid oligomers in an nose discharge sample.

### [Background Art]

A degenerative brain disease refers to a disease that occurs in the brain as it ages. As a cause unknown to date, a specific group of brain cells gradually lose their functions in the brain, it is known to be caused by death of brain nerve cells which are the most important for the communication of the brain nervous system, problems in the formation or function of synapses that transfer information between brain cells and brain cells, or an abnormal increase or decrease in the electrical activity of the brain nerve. The degenerative brain disease can be distinguished by considering the main symptoms and the affected brain region, and Alzheimer's disease (AD) can be identified by disease-specific protein expression compared to other brain diseases, death of specific cell group and specificity symptoms thereby.

Recently, Alzheimer's disease is increasing rapidly and thus there is an urgent need for early diagnosis, prevention, and treatment. To date, Alzheimer's disease can be diagnosed by only neuropsychological tests such as Short Form of Geriatric Depression Scale (GDS) or mini-mental state examination (MMSE), or specialized MRI imaging, which are time-consuming and costly methods.

For example, as an existing method for diagnosing Alzheimer's disease, there is a technique for imaging a brain using a high-resolution brain imaging apparatus. For early diagnosis of Alzheimer's disease using brain imaging, brain imaging is performed on patients with suspected Alzheimer's disease to measure the degree of abnormal accumulation of beta amyloid protein and to compare and analyze the patient's post brain tissue test results with researching the precision of brain imaging devices. However, such an image-based diagnostic method not only requires a high cost to the patient, but also detects disease in a state where brain contraction or damage has already progressed, so that the detection of the disease is delayed.

Another representative diagnostic method is to diagnose spinal fluid by measuring changes in the amount of beta amyloid protein in the cerebrospinal fluid. However, the cerebrospinal fluid analysis method itself is known to be a very painful to patients, and it is pointed out as a disadvantage that the risk is accompanied by the examination.

Under this background, the present inventors developed a method of diagnosing cranial nerve disease using nasal fluid sample collected by a non-invasive method, and disclosed it in Korean Patent Publication No. 10-2015-0144283. Specifically, the publication discloses a method of diagnosing cranial nerve disease by measuring the expression level of beta amyloid oligomer in the nasal fluid sample. However, the method can only confirm whether or not the cranial nerve disease has developed, and a complicated pretreatment process of amyloid decomposition is required in using a nasal fluid sample.

Therefore, there is still a need for a non-invasive diagnostic method that minimizes the pretreatment process of a sample and precisely diagnoses by classifying the stage of progression of cranial nerve disease as well as the development of cranial nerve disease.

### [Disclosure]

### [Technical Problem]

Accordingly, the present inventors completed the present invention by developing a method of screening the ongoing progress of Alzheimer's disease by measuring the expression levels of different types of beta amyloid oligomers in a nose discharge sample without pre-treatment of the sample.

Accordingly, an object of the present invention is to provide a composition for screening the ongoing progress of Alzheimer's disease, comprising an agent measuring the expression levels of different types of beta amyloid oligomers in a nose discharge sample.

Another object of the present invention is to provide a kit for screening the ongoing progress of Alzheimer's disease comprising the composition.

Another object of the present invention is to provide a method of screening the ongoing progress of Alzheimer's disease by measuring the expression levels of different types of beta amyloid oligomers in a nose discharge sample.

Another object of the present invention is to provide a method of screening a therapeutic agent for mild Alzheimer's disease by treating a candidate substance for treatment of mild Alzheimer's disease in a nose discharge sample isolated from a patient suffering from mild Alzheimer's disease and then measuring the expression levels of different types of beta amyloid oligomers.

Another object of the present invention is to provide a method of screening a therapeutic agent for mild Alzheimer's disease by treating a candidate substance for treatment of mild Alzheimer's disease in a nose discharge sample isolated from a patient suffering from moderate Alzheimer's disease and then measuring the expression levels of different types of beta amyloid oligomers.

Another object of the present invention is to provide a screening kit for treating mild and moderate Alzheimer's disease, comprising an agent measuring the expression levels of different types of beta amyloid oligomers in a nose discharge sample.

### [Technical Solution]

In order to solve the above-described problems, the present invention provides a composition for screening the ongoing progress of Alzheimer's disease, comprising an agent measuring an expression level of a 56 kDa beta amyloid oligomer and a 72 kDa beta amyloid oligomer in an isolated nose discharge sample.

According to a preferred embodiment of the present invention, the agent measuring an expression level of a 56 kDa beta amyloid oligomer and a 72 kDa beta amyloid oligomer may be an antibody or aptamer that specifically binds the 56 kDa beta amyloid oligomer and the 72 kDa beta amyloid oligomer.

The present invention also provides a kit for screening the ongoing progress of Alzheimer's disease comprising the above composition.

The present invention also provides a method of screening the ongoing progress of Alzheimer's disease comprising:
(a) measuring an expression level of a 56 kDa beta amyloid oligomer in an isolated nose discharge sample; and
(b) measuring an expression level of a 72 kDa beta amyloid oligomer in the sample of step (a).

According to a preferred embodiment of the present invention, if in step (a), the expression level of the 56 kDa beta amyloid oligomer is increased compared to that of a normal control group and the expression level of the 72 kDa beta amyloid is no different from that of a normal control group, it can be diagnosed as mild Alzheimer's disease.

The present invention also provides a method of diagnosing and treating mild Alzheimer's disease comprising:
(a) measuring an expression level of 56 kDa beta amyloid oligomer in the isolated nose discharge sample;
(b) measuring an expression level of 72 kDa beta amyloid oligomer of 72 kDa in the sample of step (a);
(c) diagnosing as mild Alzheimer's disease when in step (a), the expression level of the 56 kDa beta amyloid oligomer is increased compared to that of a normal control group and the expression level of the 72 kDa beta amyloid is no different from that of a normal control; and
(d) administering to a subject diagnosed with mild Alzheimer's disease an agent that inhibits the expression or activity of the 56 kDa beta amyloid oligomer.

The present invention also provides a method of screening and treating the ongoing progress of Alzheimer's disease comprising:
(a) measuring an expression level of a 56 kDa beta amyloid oligomer in an isolated nose discharge sample; and
(b) measuring an expression level of a 72 kDa beta amyloid oligomer in the sample of step (a).

According to another preferred embodiment of the present invention, if in step (a), the expression levels of the 56 kDa beta amyloid oligomer and the 72 kDa beta amyloid are increased compared to a normal control group, it is diagnosed as moderate Alzheimer's disease.

The present invention also provides a method of diagnosing and treating moderate Alzheimer's disease comprising:
(a) measuring an expression level of 56 kDa beta amyloid oligomer in the isolated nose discharge sample;
(b) measuring an expression level of 72 kDa beta amyloid oligomer in the sample of step (a);
(c) diagnosing as moderate Alzheimer's disease when in step (a), the expression levels of the 56 kDa beta amyloid oligomer and the 72 kDa beta amyloid oligomer are increased compared to that of a normal control group; and
(d) administering to a subject diagnosed with moderate Alzheimer's disease an agent that inhibits the expression or activity of the 56 kDa beta amyloid oligomer and/or an agent that inhibits the expression or activity of the 72 kDa beta amyloid oligomer.

According to another preferred embodiment of the present invention, the steps of (a) and (b) may be performed by Western blot, ELISA (enzyme linked immunosorbent assay), immunoprecipitation assay, complement fixation assay, flow cytometry (Fluorecence Activated Cell Sorter, FACS) or protein chip.

The present invention also provides a method of screening a therapeutic agent for mild Alzheimer's disease comprising:
(a) treating a candidate substance for treatment of mild Alzheimer's disease in a nose discharge sample isolated from a patient suffering from mild Alzheimer's disease;
(b) measuring an expression level of a 56 kDa beta amyloid oligomer in the sample of step (a); and
(c) measuring an expression level of a 72 kDa beta amyloid oligomer in the sample of step (b).

According to a preferred embodiment of the present invention, if an expression level of the 56 kDa beta amyloid oligomer in step (b) is reduced than that before treating the candidate substance, and an expression level of the 72 kDa beta amyloid oligomer in step (c) does not differ from that before treating the candidate substance, it can be selected as a therapeutic agent for mild Alzheimer's disease.

The present invention also provides a method of screening a therapeutic agent for moderate Alzheimer's disease comprising:
(a) treating a candidate substance for treatment of moderate Alzheimer's disease in a nose discharge sample isolated from a patient suffering from moderate Alzheimer's disease;
(b) measuring an expression level of a 56 kDa beta amyloid oligomer in the sample of step (a); and
(c) measuring an expression level of a 72 kDa beta amyloid oligomer in the sample of step (b).

According to a preferred embodiment of the present invention, if an expression level of the 56 kDa beta amyloid oligomer in step (b) is reduced than that before treating the candidate substance, and an expression level of the 72 kDa beta amyloid oligomer in step (c) is reduced than before treating the candidate substance, it can be selected as a therapeutic agent for moderate Alzheimer's disease.

The present invention also provides a kit for screening a therapeutic agent for mild or moderate Alzheimer's disease, comprising an agent for measuring an expression level of a 56 kDa beta amyloid oligomer and a 72 kDa beta amyloid oligomer in an isolated nose discharge sample.

The present invention also provides use of a composition comprising a 56 kDa beta amyloid oligomer and a 72 kDa beta amyloid oligomer for the preparation of an agent for screening the ongoing progress of Alzheimer's disease.

### [Advantageous Effects]

According to a method of screening the ongoing progress of Alzheimer's disease of the present invention, the ongoing progress of Alzheimer's disease can be screened accurately and quickly in a non-invasive manner by simultaneously identifying two beta amyloid oligomers in a nose discharge sample. In addition, by checking the form and amount of one protein without complicated pretreatment of sample, it is possible to easily and accurately screen Alzheimer's onset and stage of development and is economical in terms of cost. Therefore, the method for screening the ongoing progress of Alzheimer's disease of the present invention can greatly assist in establishing a patient-oriented and effective strategy for treating Alzheimer's disease.

### [Description of Drawings]

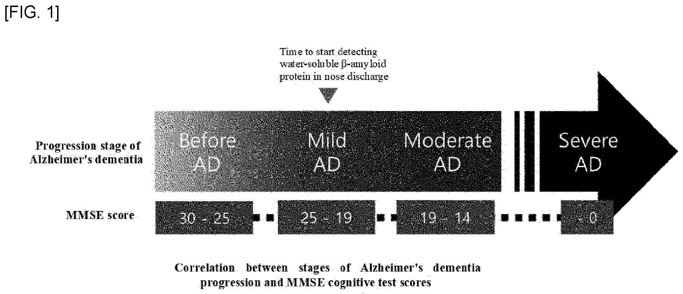

FIG. 1 is a schematic diagram showing the correlation between the stage of Alzheimer's dementia progression and the MMSE cognitive test score.

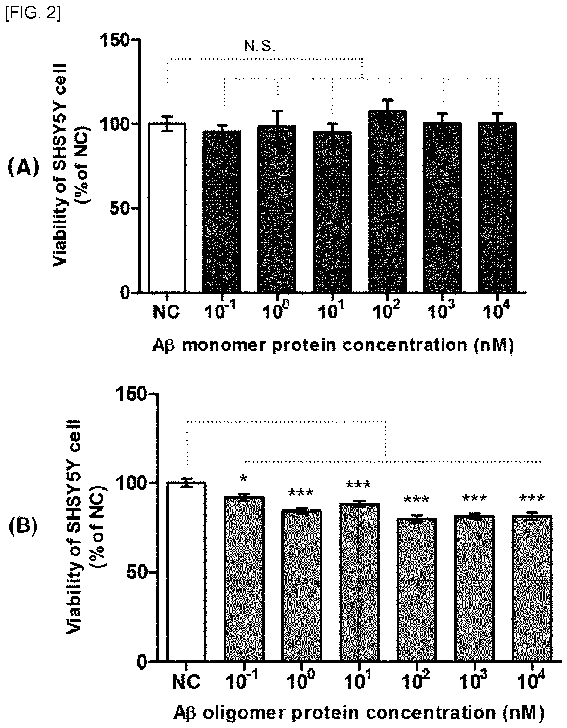

FIG. 2 is a graph showing the results of cytotoxicity test according to the treatment by concentration of beta amyloid in the form of monomer and beta amyloid in the form of oligomer (Aβ42).

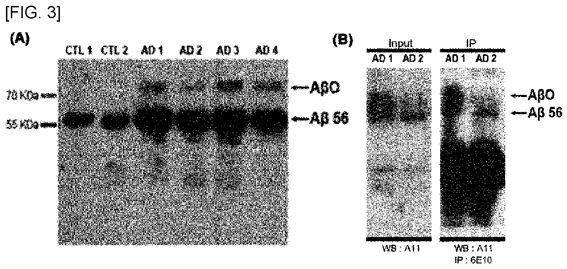

FIG. 3A shows a quantitative analysis of the target beta amyloid oligomer by Western blot in nose discharge samples taken from the normal and Alzheimer's patient groups, and FIG. 3B shows results of performing the immunoprecipitation reaction additionally using monomeric amyloid beta specific antibody (6E10) only Alzheimer's patients.

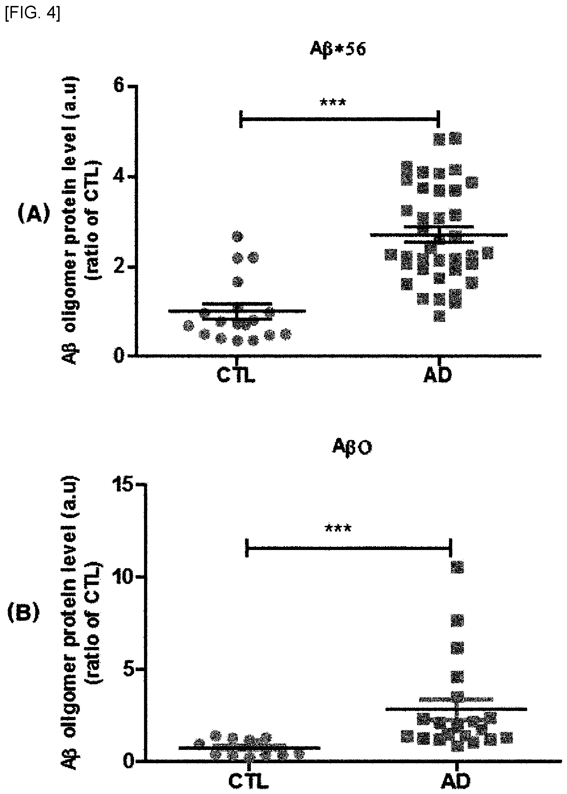

FIG. 4A and FIG. 4B are graphs showing results of measuring the expression levels of beta amyloid oligomers of 56 kDa and 72 kDa in the normal human and Alzheimer's patient groups, respectively and FIG. 4A shows the expression level of the 56 kDa beta amyloid oligomer and FIG. 4B shows the expression level of the 72 kDa beta amyloid oligomer.

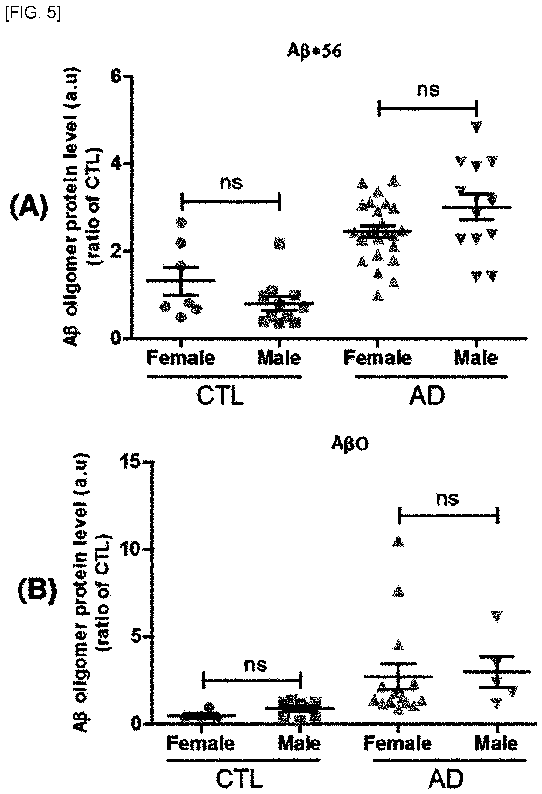

FIG. 5A and FIG. 5B are graphs showing results of measuring the expression levels of beta amyloid oligomers of 56 kDa and 72 kDa according to sex in the normal human and the Alzheimer's patient groups, respectively and FIG. 5A shows the expression level of the 56 kDa beta amyloid oligomer and FIG. 5B shows the expression level of the 72 kDa beta amyloid oligomer.

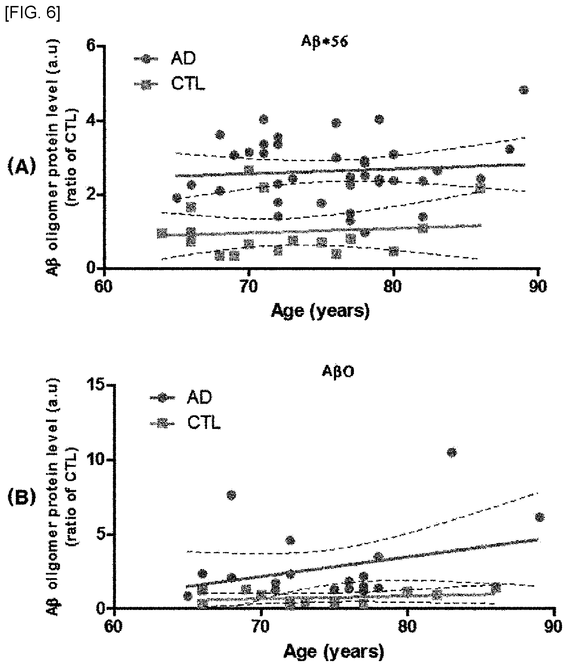

FIG. 6A and FIG. 6B are graphs showing results of measuring the expression levels of beta amyloid oligomers of 56 kDa and 72 kDa according to age in the normal human and the Alzheimer's patient groups, respectively and FIG. 6A shows the expression levels of the 56 kDa beta amyloid oligomers and FIG. 6B shows the expression level of the 72 kDa beta amyloid oligomer.

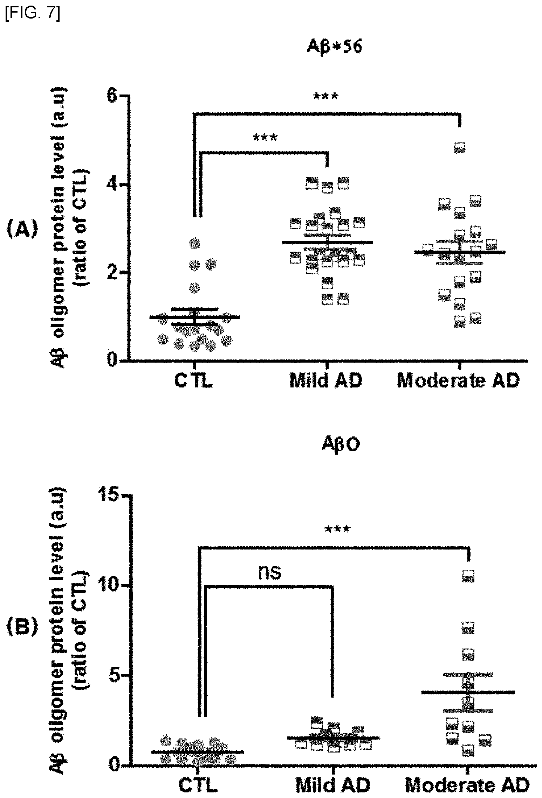

FIG. 7A and FIG. 7B are graphs showing results of measuring the expression levels of beta amyloid oligomers of 56 kDa and 72 kDa according to the Alzheimer's progression stage (mild Alzheimer and moderate Alzheimer), and FIG. 7A shows the expression level of the 56 kDa beta amyloid oligomer and FIG. 7B shows the expression level of the 72 kDa beta amyloid oligomer.

### [Best Mode]

Hereinafter, the present invention will be described in more detail.

As described above, there is still a need for a non-invasive diagnostic method capable of minimizing the pretreatment process of samples and accurately diagnosing by classifying the stage of progression of cranial nerve disease as well as the development of cranial nerve disease. Accordingly, the present inventors sought solutions to the above-mentioned problems by developing a method of screening the progression stage of Alzheimer's disease by measuring the expression levels of two different types of beta amyloid oligomers in nose discharge samples.

In the method of screening the progression stage of Alzheimer's disease of the present invention, Alzheimer's ongoing progress can be screened accurately and quickly in a non-invasive manner by simultaneously confirming two types of beta amyloid oligomers in a nose discharge sample. In addition, by checking the form and amount of one protein without complicated pretreatment of sample, it is possible to easily and accurately screen Alzheimer's onset and progression stage and is economical in terms of cost.

Accordingly, the present invention provides a composition for screening ongoing progress of Alzheimer's disease, comprising an agent measuring an expression level of a 56 kDa beta amyloid oligomer and a 72 kDa beta amyloid oligomer in an isolated nose discharge sample.

Prior art techniques for confirming Alzheimer's progress include MMSE cognitive tests (Folstein MF, Folstein S, McHugh P. Mini-mental state: a practical method for grading the cognitive state of patients for the clinician. J Psychiatr Res 1975;12: 189-98), and as shown in FIG. 1, the progression stage can be divided according to the MMSE score.

The beta amyloid oligomer polymerized with 12 or more and 20 or less has water-soluble characteristics and exhibits toxicity. In the present invention, only the 'polymerized beta amyloid oligomer' exhibiting such water solubility was targeted. Specifically, as described in the Examples, a sample of a runny nose discharge dissolved in water-soluble saline during the sample collection process was used as a target, and only the supernatant was used after centrifugation without special pretreatment.

In the present invention, the term "nose discharge sample" may include a sample of a nasal wash, for example, a sample taken with a runny nose inhaler after spraying a saline solution into the nose, a sample of a runny nose flowing through a saline solution and the like.

The beta amyloid oligomers polymerized with 12 or more and 20 or less detected from a nose discharge sample in the present invention, for example, 56 kDa and 72 kDa beta amyloid oligomers, are beta amyloid oligomers that causes cytotoxicity or cognitive impairment.

As the actual beta amyloid for measurement, the converted monomer is used, of which toxic beta amyloid and non-toxic beta amyloid cannot be distinguished. In fact, there is a conflict between the opinion that the measurement of 'beta amyloid' in the form of monomers in blood is related to Alzheimer's or not. (Refer to Toledo JB, Shaw LM, Trojanowski JQ (2013) Plasma amyloid beta measurements - a desired but elusive Alzheimer's disease biomarker. Alzheimers Res Ther 5: 8.10.1186/alzrt162 PubMed: 23470128 [PMC free article]; and Lopez OL, Kuller LH, Mehta PD, Becker JT, Gach HM et al. (2008) Plasma amyloid levels and the risk of AD in normal subjects in the Cardiovascular Health Study. Neurology 70: 1664-1671.10.1212/01.wnl.0000306696.82017.66 PubMed: 18401021). As such, prior studies showing that it is more meaningful to specifically target toxic amyloids and target oligomeric forms more than simply target the amount of beta amyloid itself have been reported.

The beta amyloid monomer derived from the amyloid precursor protein binds another monomer in the extracellular region to form a dimer, complex or oligomer. As described above, Aβ*56 (56 kDa beta amyloid oligomer) and AβO (72 kDa beta amyloid oligomer) in the oligomer form are water-soluble and exhibit expression with cognitive decline in Alzheimer's.

The accumulation of water-soluble beta amyloid oligomers between neurons interferes with synaptic formation in patients with Alzheimer's disease, and water-soluble amyloids are more detrimental to the formation of synapse than non-aqueous amyloid forms with intensified oligomer formation. It is generally known that when the oligomer formation is intensified, it is insoluble in water. As shown in FIG. 1, in the nose discharge sample used in the present invention, water-soluble beta amyloid starts to be detected from the mild Alzheimer's stage.

The present inventors confirmed that it is possible to detect a water-soluble amyloid protein having such a harmful effect in a nose discharge secreted outside the body.

To confirm whether the oligomer form of beta amyloid exhibits higher toxicity than the monomer form of beta amyloid, a toxicity test was performed using human nerve cancer cells (SHSY5Y cells) as described in Example 1. As a result, as shown in FIG. 2, the beta amyloid monomer did not show toxicity even when the treatment concentration increased, whereas the Aβ42 oligomer showed a significant decrease in cell viability as the treatment concentration increased, indicating that only the oligomer form exhibited toxicity. This means that measuring the beta amyloid oligomer can more effectively explain the toxic effect in beta amyloid-related disease progression than measuring the monomer.

As mentioned above, the present inventors have demonstrated that it is possible to diagnose cranial nerve disease by measuring the expression level of beta amyloid oligomer in a nose discharge sample in Korean Patent Publication No. 10-2015-0144283. In the present invention, an immunoprecipitation reaction was further performed using the monomer form of beta amyloid specific antibody, and it was verified that the oligomer form of beta amyloid was composed of a set of monomers (see FIG. 3).

Based on the above results, it was verified through Example 3 that a beta amyloid oligomer polymerized with 12 or more and 20 or less, such as a beta amyloid oligomer of 56 kDa or 72 kDa, can be used as a diagnostic marker for Alzheimer's disease (FIG. 4A and FIG. 4B).

In addition, in order to confirm whether the beta amyloid oligomer of 56 kDa or 72 kDa showed a difference in expression level by factors other than Alzheimer's disease, expression levels according to sex and age were measured in the normal and Alzheimer's patients, respectively. As a result, as shown in FIG. 5A-5B and FIG. 6A-6B, changes in the expression levels of beta amyloid oligomers of 56 kDa and 72 kDa according to sex and age were not observed. Through this, it was proved that both beta amyloid oligomers of 56 kDa and 72 kDa can be used as markers specific for Alzheimer's disease.

Finally, the present inventors measured the expression levels of 56 kDa and 72 kDa beta amyloid oligomers according to the stage of Alzheimer's disease progression, and as shown in FIG. 7, the 56 kDa beta amyloid oligomers were specific in both mild and moderate Alzheimer's (FIG. 7A), however the 72 kDa beta amyloid oligomer was found to be specific only to moderate Alzheimer's (FIG. 7B).

From the above results, Alzheimer's disease progresses as the form of the beta amyloid oligomer changes from 56 kDa to 72 kDa, and it can be seen that the Alzheimer's ongoing progress can be screened easily and accurately without a pre-treatment process of the sample by simultaneously measuring the expression levels of beta amyloid oligomers of 56 kDa and 72 kDa in a nose discharge sample.

In the present invention, the term "expression level of beta oligomer" refers to the level of beta oligomer formed by aggregation of monomers in a nose discharge sample, and it is possible to confirm whether the onset and progression stage of Alzheimer's disease more accurately by measuring the expression level of the oligomer, than measuring the expression level of the monomer.

In the present invention, it is possible to diagnose the development of Alzheimer's disease and screen the stage of progression of Alzheimer's disease by observing the expression levels of water-soluble and toxic beta amyloid oligomers of 56 kDa and 72 kDa in a nose discharge sample.

The term "agent for measuring the expression level of beta oligomer" of the present invention includes antibodies or aptamers that specifically bind to the target oligomer, but it is not particularly limited thereto.

The term "antibody" refers to a specific protein molecule directed against an antigenic site. For purposes of the present invention, an antibody refers to an antibody that specifically binds to a 56 kDa or 72 kDa beta amyloid oligomer, and it includes all polyclonal antibodies, monoclonal antibodies and recombinant antibodies. An antibody that specifically binds to the target oligomer can be easily prepared using techniques well known in the art.

Polyclonal antibodies can be produced by methods well known in the art in which a target protein antigen is injected into an animal and blood is collected from the animal to obtain serum containing the antibody. Such polyclonal antibodies can be produced from any animal species host, such as goat, rabbit, sheep, monkey, horse, pig, cow and dog.

A monoclonal antibody can be prepared by a hybridoma method well known in the art (see Kohler and Milstein (1976) European Journal of Immunology 6:511-519), or a phage antibody library (Clackson et al, Nature, 352: 624-628, 1991; Marks et al, J. Mol. Biol., 222:58, 1-597, 1991). Antibodies prepared by the above method can be separated and purified using methods such as gel electrophoresis, dialysis, salt precipitation, ion exchange chromatography and affinity chromatography.

In addition, the antibody of the present invention includes a complete form having two full-length light chains and two full-length heavy chains, as well as functional fragments of antibody molecules. A functional fragment of an antibody molecule means a fragment having at least an antigen-binding function, and includes Fab, F(ab'), F(ab') 2, Fv and the like.

An analysis method for confirming the amount of the target oligomer bound to the antibody using the antibody, includes Western blot, ELISA (enzyme linked immunosorbent assay), radioimmunoassay (RIA), radioimmunodiffusion, Ouchterlony Immune diffusion method, rocket immunoelectrophoresis, tissue immunostaining, immunoprecipitation assay, complement fixation assay, flow cytometry (fluorescence activated cell sorter, FACS), protein chip (protein) chip), and the like, but it is not limited thereto.

In addition, in the present invention, "aptamer" refers to single-stranded DNA (ssDNA) or RNA having high specificity and affinity for a specific substance. The aptamer has a very high and stable affinity for a specific substance, can be synthesized in a relatively simple way, modified in a variety of ways to increase their binding power, and targeted for cells, proteins, and even small organic substances and thus its specificity and stability are very high compared to the antibodies already developed.

The aptamer of the present invention is not limited as long as it can specifically bind to the beta amyloid oligomer, and the base used in the aptamer may be selected from the group consisting of A, G, C, U and bases of deoxy form thereof, unless otherwise specified.

In addition, in order to enhance stability, the aptamer may be modified by binding at least one selected from the group consisting of polyethylene glycol (PEG), idT (inverted deoxythymidine), LNA (Locked Nucleic Acid), 2'-methoxy nucleosides, 2'-amino nucleosides, 2'F-nucleosides, amine linkers, thiol linkers and cholesterol, to the 5'end site, the middle site, the 3'end site, or both ends thereof.

The idT (inverted deoxythymidine) is one of the molecules which are generally used to prevent degradation by nucleases of aptamers that are weakly resistant to nucleases. The nucleic acid unit forms a chain by bonding 3'-OH of the preceding unit and 5'-OH of the next unit, but idT combines 3'-OH of the preceding unit and 3'-OH of the next unit and it is a molecule causing the effect of inhibiting degradation by 3'exonuclease, which is a kind of nuclease, by artificially changing so that 5'-OH, not 3'-OH is exposed.

The present invention also relates to a kit for screening ongoing progress of Alzheimer's disease comprising the above-described composition.

The kit of the present invention may include an agent capable of measuring the expression level of beta amyloid oligomer of 56 kDa or 72 kDa in a nose discharge sample, one or more compositions, solutions, or devices suitable for analysis of expression levels. For example, the kit may include a substrate, an appropriate buffer, a secondary antibody labeled with a detection label, and a chromogenic substrate and the like for immunological detection of the antibody.

As an "agent measuring an expression level of a 56 kDa beta amyloid oligomer and a 72 kDa beta amyloid oligomer", as described above, an antibody or aptamer, etc. capable of specifically binding to the target oligomer may be used and the kit may include in the form of protein chip containing the agent.

Furthermore, it may further include a secondary antibody capable of detecting the antibody or aptamer, the secondary antibody is not particularly limited thereto, but it may be bound to 56 kDa or 72 kDa beta amyloid capable of binding to the protein chip and it can be labeled with fluorescent materials, radioactive isotopes, and the like.

Specifically, the kit for screening of progression stage of Alzheimer's disease may be characterized by including essential elements necessary for performing ELISA in order to implement various ELISA methods such as an ELISA kit and a sandwich ELISA. The ELISA kit can include antibodies or aptamers specific for the proteins. Antibodies may have high specificity and affinity for beta amyloid oligomers of 56 kDa or 72 kDa, respectively, and little cross reactivity to other proteins, and may be monoclonal antibodies, polyclonal antibodies or recombinant antibodies. In addition, the ELISA kit may include antibodies or aptamers specific for the control protein. Other ELISA kits may include reagents capable of detecting bound antibodies, such as labeled secondary antibodies, chromopores, enzymes, and other substances capable of binding the substrate or antibody thereof.

Besides, the kit may be a kit for implementing Western blot, ELISA (enzyme linked immunosorbent assay), immunoprecipitation assay, complement fixation assay, flow cytometry (Fluorescence Activated Cell Sorter, FACS), a protein chip, or etc., and may additionally include additional components suitable for each analysis method. Through these analysis methods, it is possible to accurately diagnose and screen the progression stage of Alzheimer's disease by comparing the amount of antigen-antibody complex formation, and to provide a patient-specific treatment method according to the progression stage.

As described above, the present invention provides a screening method and a kit capable of checking the progression step continuously and simply while simultaneously increasing the accuracy of Alzheimer's diagnosis by cross-checking the two different beta amyloid oligomers.

In addition, the present invention relates to a method of screening ongoing progress of Alzheimer's disease comprising: (a) measuring an expression level of a 56 kDa beta amyloid oligomer in an isolated nose discharge sample; and (b) measuring an expression level of a 72 kDa beta amyloid oligomer in the sample of step (a).

Specifically, if in step (a), the expression level of the 56 kDa beta amyloid oligomer is increased compared to that of a normal control group and the expression level of the 72 kDa beta amyloid oligomer is no different from that of a normal control group, it can be diagnosed as mild Alzheimer's disease.

In step a), when the expression levels of 56 kDa beta amyloid and 72 kDa beta amyloid oligomers are increased compared to the normal control group, it can be diagnosed as moderate Alzheimer's disease.

In the method of screening the progression stage of Alzheimer's disease of the present invention, the nose discharge sample may be separated from an individual to be confirmed for the diagnosis or progression stage of Alzheimer's disease, and the process of separating proteins from the nose discharge sample may be performed by using a known process and the protein level can be measured by the various antigen-antibody complex measurement methods mentioned above. However, in the present invention, the process of pre-treating a nose discharge sample confirms an oligomer-type beta amyloid as it is, which does not require a complicated process of decomposing amyloid. Also, no concentration step for measurement is necessary. As described in Example 2-3, a nose discharge sample collected using water-soluble saline was used by extracting only the supernatant after centrifugation.

Measurement of the expression level of a 56 kDa or 72 kDa beta amyloid oligomer can be performed using an antibody or aptamer that specifically binds a 56 kDa or 72 kDa beta amyloid oligomer. More specifically, the antibody may be a monoclonal antibody or a polyclonal antibody specific for a beta amyloid oligomer of 56 kDa or 72 kDa, and may be a functional fragment of an antibody having antigen-binding activity, but it is not limited thereto.

In the method of screening the progression stage of Alzheimer's disease of the present invention, Western blot, ELISA (enzyme linked immunosorbent assay), immunoprecipitation assay, complement fixation assay), flow cytometry (Fluorescence Activated Cell Sorter, FACS) or a protein chip (protein chip) may be exemplified as a method for detecting beta amyloid oligomeric protein from a nose discharge sample, but it is not limited thereto.

The present invention also relates to a method of diagnosing and treating mild Alzheimer's disease comprising:
(a) measuring an expression level of 56 kDa beta amyloid oligomer in the isolated nose discharge sample;
(b) measuring an expression level of 72 kDa beta amyloid oligomer of 72 kDa in the sample of step (a);
(c) diagnosing as mild Alzheimer's disease when in step (a), the expression level of the 56 kDa beta amyloid oligomer is increased compared to that of a normal control group and the 72 expression level of the 72 kDa beta amyloid is no different from that of a normal control; and
(d) administering to a subject diagnosed with mild Alzheimer's disease an agent that inhibits the expression or activity of the 56 kDa beta amyloid oligomer.

The present invention also provides a method of diagnosing and treating moderate Alzheimer's disease comprising:
(a) measuring an expression level of 56 kDa beta amyloid oligomer in the isolated nose discharge sample;
(b) measuring an expression level of 72 kDa beta amyloid oligomer in the sample of step (a);
(c) diagnosing as moderate Alzheimer's disease when in step (a), the expression levels of the 56 kDa beta amyloid oligomer and the 72 kDa beta amyloid oligomer are increased compared to that of a normal control group; and
(d) administering to a subject diagnosed with moderate Alzheimer's disease an agent that inhibits the expression or activity of the 56 kDa beta amyloid oligomer and/or an agent that inhibits the expression or activity of the 72 kDa beta amyloid oligomer.

As shown in FIG. 7A and FIG. 7B, the expression level of 56 kDa beta amyloid oligomer was significantly increased in both the mild Alzheimer's disease group and the moderate Alzheimer's disease group compared to that of the normal control group, but the expression level of the 72 kDa beta amyloid oligomer was increased significantly in the moderate Alzheimer's disease group compared to that of the normal control group, and there was no significant difference in the mild Alzheimer's disease group.

Therefore, an agent that suppresses the expression or activity of 56 kDa beta amyloid oligomer is administered to a patient diagnosed with a mild Alzheimer's disease, and an agent which inhibits the expression or activity of 56 kDa beta amyloid oligomer and/or an agent which inhibits the expression or activity of the 72 kDa beta amyloid oligomer is administered to a patient diagnosed with a moderate Alzheimer's disease, and thus it is possible to effectively treat mild and moderate Alzheimer's disease in patient-customized way according to the stage of progression of Alzheimer's disease.

In the diagnosis and treatment method of mild Alzheimer's disease of the present invention, a well-known therapeutic agent for mild Alzheimer's disease in step (d) may be administered.

The therapeutic agent for mild Alzheimer's disease may be, for example, donepezil (product name: Aricept), rivastigmine (product name: Exelon) or galantamine (product name: Razadyne), but it is not limited thereto.

In the method of diagnosing and treating a mild Alzheimer's disease of the present invention, an agent that suppresses the expression or activity of beta amyloid oligomer of 56 kDa to an individual diagnosed with mild Alzheimer's disease in step (d) may be used in combination with a conventional therapeutic agent for mild Alzheimer's disease.

In the method of diagnosing and treating a moderate Alzheimer's disease of the present invention, a well-known therapeutic agent for a moderate Alzheimer's disease may be administered in step (d).

The agent for treating moderate Alzheimer's disease may include, for example, memantine (product name: Namenda, Ebixa, Axura) or a complex drug of donepezil and memantine (product name: Namzaric), but it is not limited thereto.

In the method of diagnosing and treating a moderate Alzheimer's disease of the present invention, an agent that inhibits the expression or activity of a 56 kDa beta amyloid oligomer and an agent that inhibits the expression or activity of a 72 kDa beta amyloid oligomer in an individual diagnosed with a moderate Alzheimer's disease in step (d) may be are co-administered with a therapeutic agent for moderate Alzheimer's disease.

In addition, the present invention relates to a method of screening a therapeutic agent for mild Alzheimer's disease comprising:
(a) treating a candidate substance for treatment of mild Alzheimer's disease in a nose discharge sample isolated from a patient suffering from mild Alzheimer's disease;
(b) measuring an expression level of a 56 kDa beta amyloid oligomer in the sample of step (a); and
(c) measuring an expression level of a 72 kDa beta amyloid oligomer in the sample of step (b).

Specifically, if an expression level of the 56 kDa beta amyloid oligomer in step (b) is reduced than that before treating the candidate substance, and an expression level of the 72 kDa beta amyloid oligomer in step (c) does not differ from that before treating the candidate substance, it can be selected as a therapeutic agent for mild Alzheimer's disease.

In addition, the present invention relates to a method of screening a therapeutic agent for moderate Alzheimer's disease comprising:
(a) treating a candidate substance for treatment of moderate Alzheimer's disease in a nose discharge sample isolated from a patient suffering from moderate Alzheimer's disease;
(b) measuring an expression level of a 56 kDa beta amyloid oligomer in the sample of step (a); and
(c) measuring an expression level of a 72 kDa beta amyloid oligomer in the sample of step (b).

Specifically, if an expression level of the 56 kDa beta amyloid oligomer in step (b) decreases than that before treating the candidate substance, and an expression level of the 72 kDa beta amyloid oligomer in step (c) decreases than that before treating the candidate substance, it can be selected as a therapeutic agent for moderate Alzheimer's disease.

In the screening method of the therapeutic agent for treating mild or moderate Alzheimer's disease of the present invention, an increase in the expression level of the target oligomer means an increase than the protein expression level of the normal control group, and that target oligomer expression level does not differ between before and after treatment may mean that there is no significant difference compared to the normal control group.

Furthermore, the present invention relates to a kit for screening a therapeutic agent for mild or moderate Alzheimer's disease, comprising an agent for measuring an expression level of a 56 kDa beta amyloid oligomer and a 72 kDa beta amyloid oligomer in an isolated nose discharge sample.

In addition, the present invention relates to use of a composition comprising a 56 kDa beta amyloid oligomer and a 72 kDa beta amyloid oligomer for preparation of an agent for screening ongoing progress of Alzheimer's disease.

Hereinafter, the present invention will be described in more detail through examples. These examples are only intended to illustrate the present invention in more detail, and it will be apparent to those skilled in the art that the scope of the present invention is not limited by these examples according to the gist of the present invention.

### [Mode for Invention]

### [Example 1]

### Toxicity test of beta amyloid monomer and beta amyloid oligomer

### 1-1. Preparation of Aβ42 oligomer

Aβ42 oligomers were prepared according to the reference (Stine WB et al. Preparing synthetic Abeta in different aggregation states. Methods Mol Biol 2011; 670: 13-32). Briefly, Aβ42 peptide (GL biochem, Shanghai) was initially dissolved in hexafluoroisopropanol at a concentration of 1 mM. For aggregation, the peptide was resuspended in dry dimethylsulfoxide at 5 mM, and then added to Hams F-12 cell culture medium (PromoCell, Labclinics, Spain) at a final concentration of 100 mM for 24 hours at 4°C. In the case of a monomer, the above polymerization process was not performed.

### 1-2. Cell viability assay

In order to confirm the toxicity of the beta amyloid monomer and the beta amyloid oligomer prepared in Example 1-1, a cell viability assay was performed using human nerve cancer cell SHSY5Y (ATCC®, CRL-2266) as follows.

Cell viability was measured using Calcein-AM (#C3100MP, Invitrogen, USA). Cerebral cortex neurons of cultured rats smeared at 2.5 x 10⁴ cells per well in 96-well plates were treated with each Aβ42 oligomers and monomers at various concentrations (10⁻¹, 10⁰, 10¹, 10², 10³ and 10⁴ nM) and were incubated with 3 µM of Calcein-AM for 30 minutes. The intensity of the Calcein-AM signal (excitation 485 nm; emission 535 nm) was measured using a microplate reader (SpectraMax Plus 384 Microplate Reader, Molecular Devices, USA), and the results are shown in FIG. 2. As shown in FIG. 2, toxicity did not appear even when the treatment concentration of the monomer increased, whereas the cell viability decreased significantly as the treatment concentration of the Aβ42 oligomer increased, indicating that only the oligomer form exhibited toxicity.

### [Example 2]

### Identification of oligomeric beta amyloid protein in nose discharge sample

### 2-1. Subject selection

The normal control group selected persons over the age of 60 who did not meet the other exclusion criteria and normal cognitive ability on the Seoul Neuropsychological Screening Battery (SNSB), and the patients with Alzheimer's dementia were definite Alzheimer's disease patients according to the NINCDS/ADRDA diagnosis criteria and selected persons with deterioration in two or more cognitive domains, including memory, those who have been identified as having problems with their daily life due to cognitive decline, not other causes through neuropsychological examination, and those who do not meet other exclusion criteria.

Exclusion criteria include those identified as having no brain structural problems through brain imaging (MRI or CT), people without other degenerative brain diseases, people without cerebrovascular disease that may cause cognitive impairment, people without metabolic brain disease including thyroid disease, people without alcohol or drug addiction, and people without head trauma or psychiatric disorders.

### 2-2. Body fluid collection protocol of subject

At the outpatient visit, when a normal person and a patient visit once, neuropsychological and imaging tests were performed and after confirming the test results, nose discharge of the selected subject was collected. The selected subjects visited twice. In addition, the study was conducted with thorough information management by randomization and blindfolding so that information about normal people and patients is not exposed to those who collect the samples of the subjects and those who conduct the experiment.

The subject's nose discharge was collected by the following pretreatment protocol. First, after spraying a saline solution into the nose, a nose discharge was collected using a nose discharge inhaler and stored in a 50 ml tube. When the above method failed, a saline solution was flowed into the nose, and a flowing nasal solution was collected and stored in a 50 ml tube and stored in -20°C refrigerator until analysis. This pretreatment process does not involve a concentration step. In the experiment, 50 µl of the sample was sonicated for 10 seconds, and the target protein was detected through Western blot, ELISA and ELISPOT, and diagnosed through quantitative comparison.

### 2-3. Detection of beta amyloid oligomer in nose discharge sample

The expression level of beta amyloid oligomer was analyzed in the nose discharge sample of the patient collected in Example 2-2 through Western blot.

In order to perform Western blot, the patient sample, which had been stored at first, was added into a cell lysis buffer containing a protease inhibitor and reacted at 4 °C for 30 minutes. The reaction mixture was separated using a centrifuge at 13,000 rpm for 30 minutes and then only the supernatant was extracted, and proteins extracted from the tissue were separated using 12% SDS-PAGE. The separated proteins were transferred to a nitrocellulose membrane (Schleicher & Schuell, Dassel, Germany) and blocked with blocking buffer [TBS-T with 1% scheme milk (10 mM Tris-HCI, 0.15 M NaCl; pH 7.4)] at room temperature for 1 hour. After blocking, the membrane was washed 3 times for 10 minutes with TBS-T buffer and then reacted with the primary antibody. As the primary antibody, polyclonal rabbit Anti-Aβ-oligomer (Millipore AB9234) (1:1000) was mixed with TBS-T buffer containing 3% BSA and reacted at 4 °C for 6 hours, and the secondary antibody was reacted with HRP-linked anti-rabbit IgG (1:5000) at room temperature for 2 hours. Each reaction was washed three times for 15 minutes using TBS-T to remove non-specific reactions and confirm using ECL (Amersham).

As a result, as shown in FIG. 3A, the expression level of 55 kDa and 70 kDa for Alzheimer's patients (lanes 3 to 6) was stronger than normal (lanes 1 and 2). The results were re-verified through the immunoprecipitation reaction only for Alzheimer's patients under the same conditions as the left input of FIG. 3B.

Specifically, an immunoprecipitation reaction was performed using 6E10, a beta amyloid monomer-specific antibody. Specifically, 6E10 was incubated with a nose discharge sample at 4 °C overnight and mixed with 40 µl of protein G-Sepharose for 1 hour. Beads were washed twice with buffer A and twice with buffer B, and the protein was eluted by heating in 10 µl of loading SDS-PAGE buffer.

As a result, strong expression of Aβ*56, a beta amyloid oligomer of 56 kDa and AβO, a beta amyloid oligomer of 72 kDa, was observed in patients with Alzheimer No. 1 as shown in right of FIG. 3B, and strong expression of Aβ*56, a beta amyloid oligomer of 56 kDa was observed in patients with Alzheimer No. 2.

Information on the structural and biological effects of Aβ*56 and AβO proteins were shown in Table 1 below.

**[Table 1]**

| Protein | Structure | Biological effect |
|---|---|---|
| Aβ*56 | 12-mer Globulomer 56 kDaA11-positive | Cognitive impairment in positive rats (micromolar concentration) |
| AβO | 15-20-mer sphere-shaped vehicle 2-5 nmA11-positive | Cytotoxicity in human neural culture (5 µM) |

### [Example 3]

### Quantification of beta amyloid oligomers of 56 kDa and 72 kDa

As described in the reference [K.-A. Chang et al., Neurochemistry International 97 (2016)], beta amyloid oligomers of 56 kDa and 72 kDa were quantified by classifying Alzheimer's disease patient group and normal group.

Briefly, the classification criteria for normal control group include age of 65 years or older, a person having K-MMSE score of 25 or higher in the cognitive function test (KMMSE/GDS/CDR) and normal memory item (3 items-3 points) and the classification criteria for moderate Alzheimer's disease include age of 65 years or older and a person having K-MMSE of 19 points or less in the cognitive function test (KMMSE/GDS/CDR), and CDR of 1 point or higher or GDS of 3 points or higher, a person having CDR of 0.5 point and sum of box of 2.5 points or higher, a person who does not have a structural problem in the brain through brain imaging (MRI or CT) tests, a person without other degenerative brain diseases (e.g., Parkinson's disease), and a person without cerebrovascular disease that may cause cognitive impairment, a person without metabolic brain disease including thyroid disease, a person without alcohol or drug addiction, a person without head trauma or psychiatric disease. Protein quantification was performed using a known BCA quantification method, and the protein concentration was checked using only 1 ul of the sample, and the same quantity was quantified through it.

As a result, as shown in FIG. 4A and FIG. 4B, it was confirmed that the expression of beta amyloid oligomers of 56 kDa and 72 kDa significantly increased in the Alzheimer's patient group compared to the normal group.

### [Example 4]

### Verification of specificity of beta amyloid oligomers of 56 kDa and 72 kDa for Alzheimer's disease

If there is a difference in the expression level of the target oligomer due to factors other than the onset of Alzheimer's disease, it means that the target oligomer is not specific to Alzheimer's disease, and cannot be used as a specific marker for Alzheimer's disease. Therefore, in this example, it was confirmed whether the expression levels of the beta amyloid oligomers of 56 kDa and 72 kDa change according to sex and age.

### 4-1. Confirmation of expression level according to gender

It was confirmed whether the expression levels of the beta amyloid oligomers of 56 kDa and 72 kDa in the Alzheimer's disease patient group and the normal group of Example 3 were different according to gender, respectively. Protein quantification was performed in the same manner as in Example 3.

As a result, as shown in FIG. 5A and FIG. 5B, 56 kDa and 72 kDa beta amyloid oligomers did not show a difference in expression level according to sex in the Alzheimer's disease patient group and the normal group. This shows that the gender difference does not affect the amount of oligomer expression.

### 4-2. Confirmation of expression level according to age

In the Alzheimer's disease patient group and the normal group of Example 3, it was confirmed whether the expression levels of the beta amyloid oligomers of 56 kDa and 72 kDa were different according to age. Protein quantification was performed in the same manner as in Example 3.

As a result, as shown in FIG. 6, both the beta amyloid oligomers of 56 kDa and 72 kDa did not show a difference in expression level according to age increase in the Alzheimer's disease patient group and the normal group. This shows that age does not affect the amount of oligomer expression.

### [Example 5]

### Identification of beta amyloid oligomer expression of 56 kDa and 72 kDa according to progression stage of Alzheimer's disease

As indicated in the reference [K.-A. Chang et al., Neurochemistry International 97 (2016)], using cognitive tests to classify into normal control (Normal), mild Alzheimer's disease patient group (Mild AD) and moderate Alzheimer's disease patient group (Moderate AD) and the expression levels of beta amyloid oligomers of 56 kDa and 72 kDa according to the progression stage of Alzheimer's disease was confirmed to show the results in FIG. 7.

At this time, the classification criteria of the normal control group and moderate Alzheimer's patients are as described in Example 3, the mild Alzheimer's patient group belongs to a person at 65 years of age or older and having 25-20 points of the K-MMSE in the cognitive function test (KMMSE/GDS/CDR), a CDR of 0.5-1 or a GDS of 2-3 or more, a person who does not have structural problems in the brain through brain imaging (MRI or CT) tests, a person without other degenerative brain diseases (e.g. Parkinson's disease), a person without cerebrovascular disease that can cause cognitive impairment, a person without metabolic brain disease including thyroid disease, a person without alcohol or drug addiction, a person without head trauma or psychiatric disease.

As shown in FIG. 7A, the expression level of the 56 kDa beta amyloid oligomer was significantly increased in both the mild Alzheimer's disease group and the moderate Alzheimer's disease group compared to the normal control group. This means that the 56 kDa beta amyloid oligomer is specific to both the mild Alzheimer's disease group and the moderate Alzheimer's disease group.

In addition, as shown in FIG. 7B, the expression level of the 72 kDa beta amyloid oligomer was significantly increased only in the moderate Alzheimer's disease group compared to the normal control group, and there was no significant difference in the mild Alzheimer's disease group. This means that the 72 kDa beta amyloid oligomer is specific only to the moderate Alzheimer's disease group.

Through the above results, it can be seen that the specificity of Alzheimer's at different stages of progression varies according to the beta amyloid oligomer type, and the Alzheimer's progression stage appears as the oligomer form in the nose discharge sample changes (56 kDa→72 kDa). Therefore, when the expression levels of 56 kDa and 72 kDa beta amyloid oligomers are measured together, it is possible to accurately diagnose not only the diagnosis of Alzheimer's disease, but also the progression stage of disease, and to give helps a lot for establishing an effective patient-oriented treatment strategy for Alzheimer's disease.

### [Industrial Applicability]

In according to the method of screening the progression stage of Alzheimer's disease of the present invention, Alzheimer's progression can be screened accurately and quickly in a non-invasive manner by simultaneously confirming the two types of beta amyloid oligomers in a nose discharge sample. In addition, by checking the form and amount of one protein without complicated pretreatment of sample, it is possible to easily and accurately screen Alzheimer's onset and stage of development and is economical in terms of cost. Therefore, the method for screening the ongoing progress of Alzheimer's disease of the present invention can greatly assist in establishing a patient-oriented and effective strategy for treating Alzheimer's disease.

## Claims

1. A composition for screening ongoing progress of Alzheimer's disease, comprising an agent measuring an expression level of a 56 kDa beta amyloid oligomer and a 72 kDa beta amyloid oligomer in an isolated nose discharge sample.

2. The composition for screening ongoing progress of Alzheimer's disease of claim 1, wherein the agent measuring an expression level of a 56 kDa beta amyloid oligomer and a 72 kDa beta amyloid oligomer is an antibody or aptamer that specifically binds the 56 kDa beta amyloid oligomer and the 72 kDa beta amyloid oligomer.

3. A kit for screening ongoing progress of Alzheimer's disease, comprising the composition of claim 1 or 2.

4. A method of screening ongoing progress of Alzheimer's disease comprising:
(a) measuring an expression level of a 56 kDa beta amyloid oligomer in an isolated nose discharge sample; and
(b) measuring an expression level of a 72 kDa beta amyloid oligomer in the sample of step (a).

5. The method of screening ongoing progress of Alzheimer's disease of claim 4, wherein if in step (a), the expression level of the 56 kDa beta amyloid oligomer is increased compared to that of a normal control group and the expression level of the 72 kDa beta amyloid oligomer is no different from that of a normal control group, it is diagnosed as mild Alzheimer's disease.

6. The method of screening ongoing progress of Alzheimer's disease of claim 4, wherein if in step (a), the expression levels of the 56 kDa beta amyloid oligomer and the 72 kDa beta amyloid oligomer are increased compared to that of a normal control group, it is diagnosed as moderate Alzheimer's disease.

7. The method of screening ongoing progress of Alzheimer's disease of claim 4, wherein the steps of (a) and (b) are performed by Western blot, ELISA (enzyme linked immunosorbent assay), immunoprecipitation assay, complement fixation assay, flow cytometry (Fluorecence Activated Cell Sorter, FACS) or protein chip.

8. A method of screening a therapeutic agent for mild Alzheimer's disease comprising:
(a) treating a candidate substance for treatment of mild Alzheimer's disease in a nose discharge sample isolated from a patient suffering from mild Alzheimer's disease;
(b) measuring an expression level of a 56 kDa beta amyloid oligomer in the sample of step (a); and
(c) measuring an expression level of a 72 kDa beta amyloid oligomer in the sample of step (b).

9. The method of screening a therapeutic agent for mild Alzheimer's disease of claim 8, wherein if an expression level of the 56 kDa beta amyloid oligomer in step (b) is reduced than that before treating the candidate substance, and an expression level of the 72 kDa beta amyloid oligomer in step (c) does not differ from that before treating the candidate substance, it is selected as a therapeutic agent for mild Alzheimer's disease.

10. A method of screening a therapeutic agent for moderate Alzheimer's disease comprising:
(a) treating a candidate substance for treatment of moderate Alzheimer's disease in a nose discharge sample isolated from a patient suffering from moderate Alzheimer's disease;
(b) measuring an expression level of a 56 kDa beta amyloid oligomer in the sample of step (a); and
(c) measuring an expression level of a 72 kDa beta amyloid oligomer in the sample of step (b).

11. The method of screening a therapeutic agent for moderate Alzheimer's disease of claim 10, wherein if an expression level of the 56 kDa beta amyloid oligomer in step (b) decreases than that before treating the candidate substance, and an expression level of the 72 kDa beta amyloid oligomer in step (c) decreases than that before treating the candidate substance, it is selected as a therapeutic agent for moderate Alzheimer's disease.

12. A kit for screening a therapeutic agent for mild or moderate Alzheimer's disease, comprising an agent for measuring an expression level of a 56 kDa beta amyloid oligomer and a 72 kDa beta amyloid oligomer in an isolated nose discharge sample.

13. Use of a composition comprising a 56 kDa beta amyloid oligomer and a 72 kDa beta amyloid oligomer for preparation of an agent for screening ongoing progress of Alzheimer's disease.
